# EUROPEAN PATENT SPECIFICATION

(11) **EP 2 052 756 B1**
(45) Date of publication and mention of the grant of the patent: **28.12.2011**
(21) Application number: 07118968.2
(22) Date of filing: 22.10.2007
(51) Int. Cl.: A61M 25/09

(54) **A medical guide wire torque and method of using the torque**
Drehmomentvorrichtung für einen medizinischen Führungsdraht und Verwendung der Vorrichtung
Couple de guide-fil médical, et son procédé d'utilisation

(43) Date of publication of application: 29.04.2009
(73) Proprietor: EG Tech I/S, 4640 Faxe (DK)
(72) Inventor: Schmidt-Sørensen, Esko Jens, 4720, Præstø (DK)
(74) Representative: Holme Patent A/S

(56) References cited:
- US-A- 4 858 810
- US-A1- 2003 225 395
- US-A1- 2005 070 820
- US-B1- 6 312 404
- US-B1- 6 533 772

## Description

The present invention relates to a medical guide wire torque comprising an elongated body defining a longitudinal axis and provided with means for receiving the guide wire, which means includes a through-going bore extending inside the elongated body and accommodating a clamping body for in response to application of an external force in a first position securing the torque on the guide wire and in a second position releasing the torque from the guide wire, the wall of the elongated body has a traversing opening, an actuation means is arranged in the traverse opening in contact with the clamping body for actuating the clamping body between the first and second positions, and where the through-going bore has a section of enlarged cross-sectional area and that the clamping body is provided in said section.

The invention also relates to a method of using the medical guide wire torque.

Diagnostic procedures, such as e.g. angiography and surgical procedures for placing endovascular devices, such as stents to obliterate aneurysms or alleviate occlusive diseases, involve the use of medical guide wires. Intubation of arteries or other kinds of vessels are however often difficult to carry out because the medical guide wire needs to be moved forth and back and rotated inside the vessel in order to reach the desired position without creating damages. Hence, a high degree and freedom of directional manipulation of the medical guide wire is very important to both surgeon and patient. Various kinds of medical guide wire torques, which the surgeon can manipulate using only one hand, are suggested in the art.

US patent no. US 6,030,349 discloses a medical guide wire torque composed of an elongated main body provided with a longitudinal, extending recess for receiving the guide wire. The main body has a vertically extending hole in which a button is displaceable located. When the button is depressed the part of the guide wire extending in the recess is sagged and displaced beyond the longitudinal axis of the main body to prevent lengthwise movement of the torque along the guide wire. Each time the button is depressed sharp edges and bends are made on the guide wire. As a result, the guide wire is permanently damaged which makes further advancement of the guide wire towards a target inside the vessel difficult, as well as the bends may damage the vessel wall.

Another torque for one-hand use is known from US patent no. 5,392,778. This known torque consists of an elongated main body terminating in a set of flexible prongs. These prongs are received in a tubular body, which is arranged concentric around the main body at the guide wire exit end. The main body has an annular locking protrusion which engages a corresponding annular recess on the tubular body in which engaged position the prongs are spread apart. The forward longitudinal, axial displacement of the tubular body in relation to the main body disengages the engagement of the protrusion and the recess, and forces the prongs towards each other in a tweezers-like manner around the guide wire. This known device is highly sensible to unintentional action of external forces. If for example the tip of the tubular body hits something or is unintentionally touched by the surgeon, unintentional loosening of the tweezing force is likely to occur. Moreover, the contact area between the prongs and the guide wire is very small.

US 6,533,772 relates to a guide wire torque as defined in the preamble of claim 1.

In a first aspect according to the present invention is provided a medical guide wire torque of the kind mentioned in the opening paragraph which facilitates selective placement on a guide wire in a feasible and effective way.

In a second aspect according to the present invention is provided a medical guide wire torque of the kind mentioned in the opening paragraph which is easy to torque using only one hand.

In a third aspect according to the present invention is provided a medical guide wire torque of the kind mentioned in the opening paragraph which does not damage the medical guide wire during use.

In a fourth aspect according to the present invention is provided a medical guide wire torque of the kind mentioned in the opening paragraph which can be used for medical guide wires of different diameters.

In a fifth aspect according to the present invention is provided a medical guide wire torque of the kind mentioned in the opening paragraph which is easy and inexpensive to manufacture.

The novel and unique features whereby this is achieved is the fact that the clamping body comprises a clamping body comprising two opposed, elongated parallel wall parts diverging from each other towards the opening to define a gap for receiving the guide wire..

The actuation means serves advantageously for applying an external force at least transverse to the longitudinal axis to trigger the clamping body to act on the guide wire, to either hold on firmly to the guide wire or allow the guide wire to pass freely though the clamping body.

A firm hold of the medical guide wire torque on the guide wire is required when the surgeon needs to advance a distance of the guide wire further inside the vessel. In this first, secured position of the clamping body on the guide wire the torque also serves as a handy manipulation grip, which also allows controlled rotation of the guide wire and steering of the tip of the guide wire.

In the second, released position of the clamping body the medical guide wire torque is set free. The medical guide wire torque is moved backwards on the guide wire and repositioned at any suitable location, and the surgeon is able to advance a further length of the guide wire into the desired intubated vessel.

The torque advantageously serves for enlarging the diameter of the guide wire so that the surgeon has something to hold on when manipulating the guide wire.

the clamping surfaces of the clamping body can be given a large contact area for the guide wire.

Due to the diverging wall parts the distance of the gap decreases in the direction away from the through-opening in the elongated body enabling the use of the medical guide wire torque on a wide range of guide wire diameters without compromising the clamping force. Hence, a very thin diameter guide wire may be located deeper in the gap than a thicker guide wire. The opposed, elongated parallel wall parts clamps the various sized guide wire in-between them in a safe and strong manner.

The opposed walls may or may not be interconnected opposite the traverse opening. In other words the clamping body may be constructed as a single unit consisting of two hinged wall parts or be arranged in the internal cavity as two single wall part units in close proximity opposite the traverse opening. Each embodiment has its advantages.

In case of a single unit the entire clamping body can be made of the same material and is very easy to manufacture and mount inside the elongated body. By choosing a material, which in itself is flexible, the clamping body can be designed with integrated flexible memory. When e.g. an external force is applied to the actuation means to displace said actuation means lengthwise of and at greater distance from the longitudinal axis in order to shift from the second position to the first position the clamping body automatically reassumes its initial shape and promptly takes a firm clamping grip on the guide wire. Moreover, the hinged relationship of the two wall parts can be utilized to prevent the walls from mutual axial displacement and/or offsetting in response to relocation of the medical guide wire torque on the guide wire.

In case the clamping body consists of two separate wall parts, the walls of the clamping body can be given different properties, by selective choice of material. As an example the wall can be made with different frictional gripping abilities.

In any of the embodiments the surfaces of the gap between the wall parts engaging the guide wire may be made with a pattern for improved engagement.

In an advantageous embodiment according to the present invention the medical guide wire torque further comprises a spring means for forcing the elongated walls of the clamping body towards each other to hold the guide wire firmly in the first position. Such a spring means may be provided whether or not the wall parts of the clamping body are connected to each other.

Preferably the spring means may be a substantially U-shaped spring, which engages at least the part of the clamping body opposite the traverse opening.

Alternative the spring means may be an integrated part of the clamping body, for example by embedding the spring means inside a clamping body having interconnected wall part with no memory shape.

When the actuation means is an actuation button extending through the traverse opening an external force can be transferred to the clamping body in a particular easy way.

The actuation means may expediently be sized to allow said means to be displaceable in at least one of the directions substantially along the longitudinal axis of the elongated body and substantially perpendicular to the longitudinal axis of the elongated body. When the actuation means is depressed the gap between the opposing walls is forced open, the clamping body is in its second position, and the guide wire is set free of the medical guide wire torque to move freely in the through-going bore. When the medical guide wire torque subsequently needs to be secured onto the guide wire, to lock the guide wire inside the clamping body in the first position, the actuation means may be axially, lengthwise displaced which triggers the actuation means to move perpendicularly away from the longitudinal axis of the elongated body. A very good contact and grip between the fingers and the medical guide wire torque may be achieved if the gripping part has an uneven exterior surface.

At least the opposing faces of the opposed wall parts of the clamping body has a coefficient of friction of at least 0,02, preferably at least 0,03. It has been found that this value provides a frictional contact between the surface of the guide wire and the clamping body which allows unobstructed intentional movement of the torque along the length of the guide wire in the second position of the clamping body, and a firm engagement between the guide wire and the clamping body when the clamping body is in the first position, which engagement allow for safe manoeuvring and location of the guide wire inside the vessel.

A method of using the medical guide wire torque is also disclosed, which method comprises the steps of positioning a guide wire in the through-going bore of the elongated body, and activating the actuation means to control the clamping force of the clamping body on the guide wire. The method further comprises any of the steps of displacing the torque in the second position of the clamping body and rotating the torque fixed on the guide wire in the first position of the clamping body. These further steps of displacing and/or rotating the clamped guide wire expediently accomplish manoeuvring of the guide wire inside the vessel.

The invention will described in further details below with reference to the accompanying drawing in which
fig. 1 shows a perspective view of a first embodiment of a medical guide wire torque according to the present invention,
fig. 2 shows in part, a longitudinal sectional view taken along the line II-II in fig. 1,
fig. 3 shows a cross-sectional view taken along line III-III in fig. 1 wherein the clamping body is in its first position, and
fig. 4 shows a cross-sectional view taken along line IV-IV in fig. 1 wherein the clamping body is in its second position.

The dimensions and geometrical shape of the medical guide wire torque are in the figures shown by way of example. Within the scope of the present invention the elongated body and the actuation means may be given any suitable exterior design.

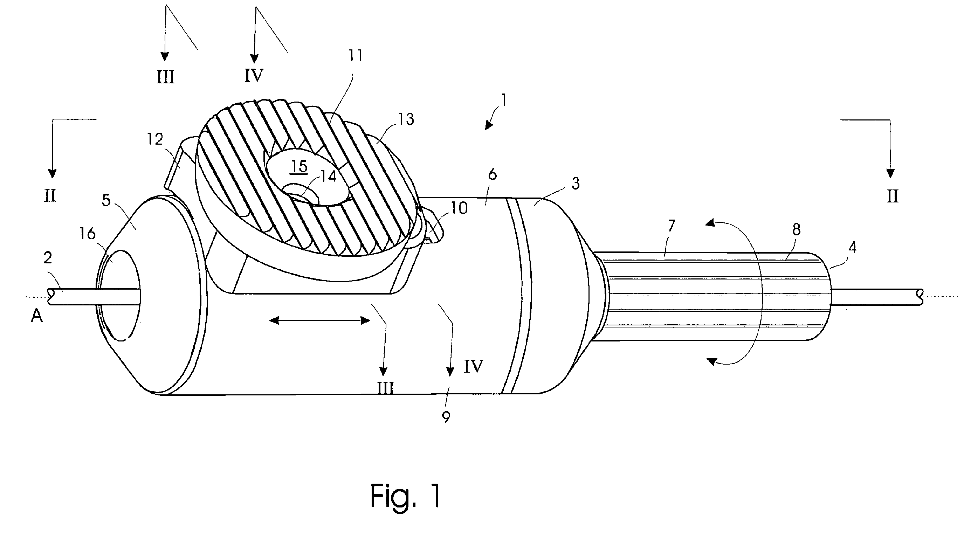

Fig. 1 shows the medical guide wire torque 1 mounted on a guide wire 2.

The torque 1 is composed of an elongated body 3 having an inlet end 4 for a guide wire 2 and an opposing exit end 5 for the guide wire. The elongated body 3 has a main body part 6, which extends into a gripping part 7 of reduced cross-section. The gripping part 7 is provided with an uneven surface in the form of circumferential spaced apart recesses 8. The recesses provide the exterior surface of the gripping part 7 with a knurled surface that enhances gripping force when the torque 1 is to be rotated as indicated with the arched arrow around the gripping part. Any other kind of surface that improves gripping force is anticipated within the scope of the present invention. For example the surface may be provided with a silicone coating for enhancing frictional force between the fingers and the gripping part 7.

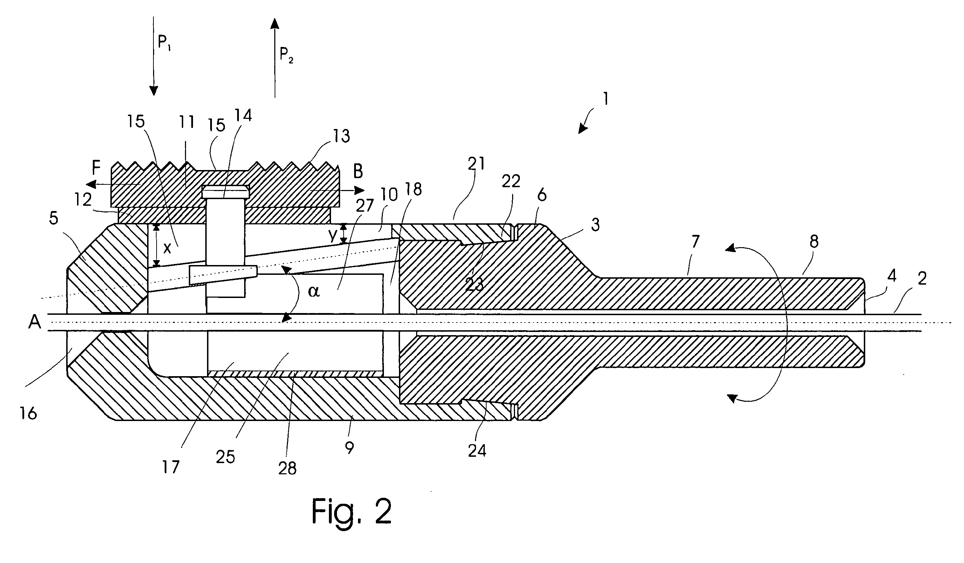

The main body part 6, which tapers towards the exit end 5, is defined by a circumferential wall 9 having a traverse opening 10 through which the actuation means 11 extends, as will be explained in more detail with reference to fig. 2.

The actuation means 11 includes a slider 12 provided with a fingerplate 13 and a wedge means 14. The fingerplate 13 has, in the embodiment shown, a cavity 15 for accommodating a fingertip so that at good grip of the slider can be obtained. The cavity 15 is optional and other means that improves the degree of contact between the finger and the fingerplate 13 may be used.

A through-going bore 16 extends along the longitudinal axis A inside the elongated body 3.

Fig. 2 shows a sectional view taken along the longitudinal axis of the torque 1 shown in fig. 1 with a guide wire 2 inserted. The guide wire 2, the wedge means 14 and one wall part of the clamping body 17 are illustrated in full.

The traverse opening 10 opens into a lengthwise hollow space 18 inside the main body 6. This hollow space 18 has a cross-sectional area, which allows the clamping body 17 to be inserted in communication with the actuation means 11 through the traverse opening 10. The inside face of the wall 9 of the hollow space 18 has opposing guide means 19a,19b for engaging corresponding arms 20a,20b on the wedge means 14.

The elongated body 3 may be moulded or otherwise made as one single integrated part into which the clamping body 17 is inserted, e.g. via the traverse opening 10. Alternatively, the clamping body is inserted during the manufacturing process. In the embodiment shown in fig. 1 and 2 the annular end 21 of the main body 6 opposite the exit end 5 is provided with an internal circumferential protrusion 22 designed to snap fit into a corresponding external circumferential recess 23 on the end 24 of the gripping part 7 opposite the inlet end 4. In this embodiment the clamping body 17 is mounted inside the hollow space 18 before the gripping part is united with the main body 6.

The guide means 19 is in the embodiment shown in fig. 1 and 2 made as lengthwise, angled slots 19 along the interior face of the wall 9. The slots 19 are arranged in an acute angle α in relation to the longitudinal axis seen from the exit end 5. This means that the distance between the slot and the longitudinal axis at the exit end 5 is smaller than the distance at the annular end 21 of the main body 6.

Various alternative guide means can, within the scope of the present invention, be used instead of slots. The same effect can e.g. be obtained by adjusting the thickness of the wall 9 at the traverse opening so that the thickness is largest at the exit end 5. In the embodiment the actuation means may advantageously further comprise a compression spring inserted between the slide 12 and the wall 9, to assist the return movement of the wedge means after the wedge means has been depressed into the clamping body 17. In yet an alternative embodiment a slot-like alternative can be made by arranging an angled protruding rail along the interior wall of the bore.

Irrespective of which guide means 19 that are used these guide means provides for the actuation means 11, in particular the wedge means 14, to move along the longitudinal axis A of the elongated body 3 while at the same time varying the radial distance of the actuation means 11 to the longitudinal axis A. As a result of this design, moving of the actuation means towards the exit end 5, i.e. in the forward direction indicated with the arrow F, forces the wedge means 14 down into the gap 27 between the opposing wall parts 25,26 of the clamping body 17 to spread apart these wall parts 25,26 and open the gap 27 to set the guide wire 2 free of the clamping force exerted by the clamping body 17. By return movement of the actuation means 11 in the direction B, the wedge means 14 is moved away from the gap 27, which triggers the opposing wall parts 25,26 to move against each other and clamp around the guide wire 2.

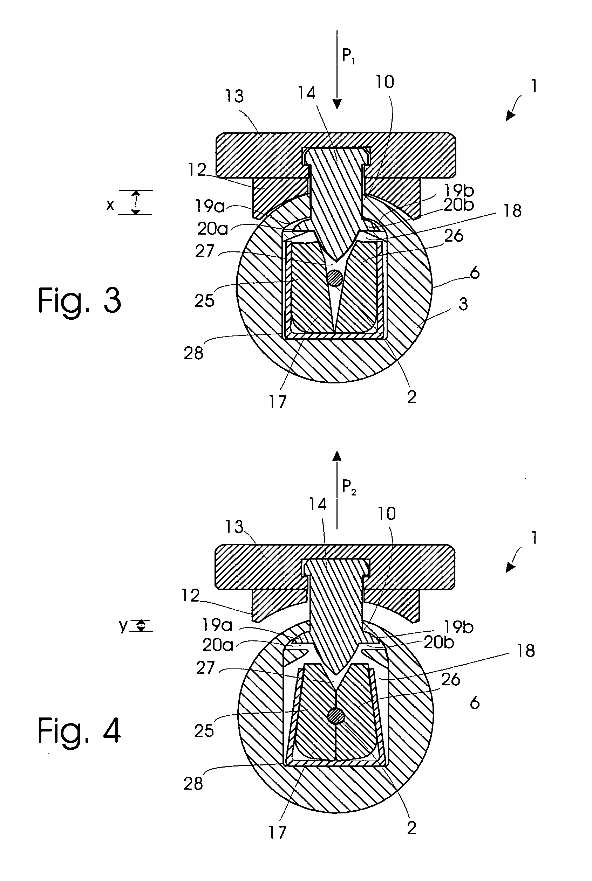

These two situations are seen more clearly in figs. 3 and 4.

Fig 3 shows a cross-sectional view taken along line III-III in fig. 2 illustrating the actuation means 11 in the second position of the clamping body 17 in which the torque 1 is fixed and secured to the guide wire 2 in a non-displaceable manner so that the tip of the guide wire is allowed to be moved both lengthwise and rotational using only one hand to reach a target area inside a vessel.

Fig. 4 shows a cross-sectional view taken along line IV-IV in fig. 2 illustrating the actuation means 11 in the first position of the clamping body 17 in which the torque 1 is not fixed and secured to the guide wire 2. In this first position of the clamping body 17 the torque 1 can be moved freely along the guide wire 2 using only one hand to reposition the torque 1 on the guide wire 2.

As is clear from fig. 3 and 4 a spring means 28 in the form of a U-shaped spring clamp 28 is arranged around the clamping body 17. This spring clamp 28 applies a spring force to the wall parts 25,26, which spring force holds the wall parts 25,26 in close proximity in the first position of the clamping body 17 where the wedge means 14 are out of the gap 27 between the wall parts 25,26, that is to say when the wedge means are moved or positioned in the direction indicated with the arrow P₂. When the wedge means are forced down, as indicated by the arrow P₁, into the gap between the wall parts 25,26 of the clamping body 17 the legs of the spring clamp are forced apart and tensioned to that once the wedge means 17 again are free of the clamping body the legs of the spring clamp returns to the position shown in fig. 4, i.e. the first position of the clamping body 17.

The wedge means can be forced down into the gap 17 between the wall parts 25,25 be simply depressing the actuation means 11 in a direction P₁ perpendicular to the longitudinal axis A, using e.g. the thumb and holding the torque 1 in the same hand. Hence, lengthwise movement is not always required for shifting from the first position to the second position of the clamping body.

The torque according to the present invention is operated different than known torques in which depression of a button locks the guide wire inside the device.

The medical guide wire torque according to the present invention and the operating principle and technique constitutes a preferred alternative to known torques. The novel torque is very easy to use using only one hand; it is reliable and does not kink the guide wire.

## Claims

1. A medical guide wire torque (1) comprising
- an elongated body (3) defining a longitudinal axis (A) and provided with means for receiving the guide wire (2),
- which means include a through-going bore (4) extending inside the elongated body (3) and a clamping body (17) for in response to application of an external force in a first position securing the torque (1) on the guide wire (2) and in a second position releasing the torque (1) from the guide wire (2),
- the wall (9) of the elongated body (3) has a traversing opening (10),
- an actuation means (11) is arranged in the traverse opening (10) in contact with the clamping body (17) for actuating the clamping body (17) between the first and second positions, and where the through-going bore (4) has a section (18) of enlarged cross-sectional area and that the clamping body (17) is provided in said section (18),
**characterised in that** the clamping body (17) comprises two opposed, elongated parallel wall parts (15,16) diverging from each other towards the traverse opening (10) to define a gap (27) for receiving the guide wire (2).

2. A medical guide wire torque (1) according to claim 1, **characterised in that** the opposed, elongated parallel wall parts (25,26) is interconnected opposite the traverse opening (10).

3. A medical guide wire torque (1) according to claims 1 or 2, **characterised in that** the clamping body (17) further comprises a spring means (28) for forcing the elongated walls (25,26) of the clamping body (17) towards each other.

4. A medical guide wire torque (1) according to claim 3. **characterised in that** the spring means (28) is a U-shaped spring (28), which engages at least a part of the clamping body (17) opposite the traverse opening (10).

5. A medical guide wire torque (1) according to any of the claims 3 or 4, **characterised in that** the spring means (28) is an integrated part of the clamping body (17).

6. A medical guide wire torque (1) according to any of the preceding claims 1 - 5, **characterised in that** the actuation means (11) is an actuation button (14) extending through the traversing opening (10).

7. A medical guide wire torque (1) according to claim 6, **characterised in that** the actuation button is a wedge means (14).

8. A medical guide wire torque (1) according to any of the preceding claims 1 - 7, **characterised in that** the actuation means (11) is displacable in at least one of the directions (F,B) substantially along the longitudinal axis of the elongated body and substantially perpendicular (P₁,P₂) to the longitudinal axis (A) of the elongated body (3).

9. A medical guide wire torque (1) according to any of the preceding claims 1 - 8, **characterised in that** the elongated body (3) has a gripping part (7) of reduced diameter, preferably said gripping part (7) has an uneven exterior surface.

10. A medical guide wire torque (1) according to any of the preceding claims 1 - 9, **characterised in that** at least the opposing faces of the opposed wall parts (25,26) of the clamping body (17) has a coefficient of friction of at least 0,02, preferably at least 0,03.

11. A method of using the medical guide wire torque (1) according to any of the preceding claims 1 - 10, wherein the method comprises the steps of
- positioning a guide wire (2) in the through-going bore (4) of the elongated body (3), and
- activating the actuation means (11) to control the clamping force of the clamping body (17) on the guide wire (12).

12. A method according to claim 11 wherein the method further comprises any of the steps of displacing the torque (1) in the second position of the clamping body (17) and rotating the torque (1) fixed on the guide wire (2) in the first position of the clamping body (17).

## Patentansprüche

1. Dreher (1) für einen medizinischen Führungsdraht, mit
- einem länglichen Körper (3), der eine Längsachse (A) definiert und mit Mitteln zum Aufnehmen des Führungsdrahts (2) versehen ist, wobei
- die Mittel eine durchgehende Bohrung (4) aufweist, die sich im Inneren des länglichen Körpers (3) erstreckt, und einen Klemmkörper (17) zum Sichern des Drehers (1) an dem Führungsdraht (2) in Antwort auf die Aufbringung einer externen Kraft in einer ersten Position und Freigeben des Drehers (1) von dem Führungsdraht (2) in einer zweiten Position,
- die Wand (9) des länglichen Körpers (3) eine Queröffnung (10) hat,
- ein Betätigungsmittel in der Queröffnung (10) in Kontakt mit dem Klemmkörper (17) zum Betätigen des Klemmkörpers (17) zwischen der ersten und der zweiten Position angeordnet ist und wobei
- die Durchgangsbohrung (4) einen Abschnitt (18) mit vergrößerten Querschnittsbereich hat und der Klemmkörper (17) in diesem Abschnitt (18) vorgesehen ist,
**dadurch gekennzeichnet, dass** der Klemmkörper (17) zwei gegenüberliegende, gestreckte, parallele Wandteile (15, 16) aufweist, die voneinander in Richtung auf die Queröffnungen (10) zur Bildung eines Spalts (17) zum Aufnehmen des Führungsdrahts (2) divergieren.

2. Ein Dreher für einen medizinischen Führungsdraht nach Anspruch 1, **dadurch gekennzeichnet, dass** die einander gegenüberliegenden, längs verlaufenden parallelen Wandteile (25, 26) der Queröffnung (10) gegenüberliegend miteinander verbunden sind.

3. Ein Dreher (1) für einen medizinischen Führungsdraht nach Anspruch 1 oder 2, **dadurch gekennzeichnet, dass** der Klemmkörper (17) weiter eine Feder (28) zum Zwingen der länglichen Wände (25, 26) des Klemmkörpers (17) aufeinander zu aufweist.

4. Ein Dreher (1) für einen medizinischen Führungsdraht nach Anspruch 3, **dadurch gekennzeichnet, dass** die Feder (28) eine U-förmige Feder (28) ist, die wenigstens einen Teil des Klemmkörpers (17) der Queröffnung (10) gegenüberliegend ergreift.

5. Ein Dreher (1) für einen medizinischen Führungsdraht nach einem der Ansprüche 3 oder 4, **dadurch gekennzeichnet, dass** das Federmittel (28) ein integrierter Bestandteil des Klemmkörpers (17) ist.

6. Ein Dreher (1) für einen medizinischen Führungsdraht nach einem der vorangehenden Ansprüche 1 - 5, **dadurch gekennzeichnet, dass** das Betätigungsmittel (11) ein Betätigungsknopf (14) ist, der sich durch die Queröffnung (10) erstreckt.

7. Ein Dreher (1) für einen medizinischen Führungsdraht nach Anspruch 6, **dadurch gekennzeichnet, dass** der Betätigungsknopf ein Keilmittel (14) ist.

8. Ein Dreher (1) für einen medizinischen Führungsdraht nach einem der vorangehenden Ansprüche 1 - 7, **dadurch gekennzeichnet, dass** das Betätigungsmittel (11) in wenigstens eine der Richtungen (F, B) im Wesentlichen entlang der Längsachse des länglichen Körpers und im Wesentlichen senkrecht (P₁, P₂) zu der Längsachse (A) des länglichen Körpers (3) verlagerbar ist.

9. Ein Dreher (1) für einen medizinischen Führungsdraht nach einem der vorangehenden Ansprüche 1 - 8, **dadurch gekennzeichnet, dass** der längliche Körper (3) ein Greifteil (7) mit verringertem Durchmesser hat, wobei der Greifteil (7) vorzugsweise eine unebene äußere Fläche hat.

10. Ein Dreher (1) nach einem der vorangehenden Ansprüche 1 - 9, **dadurch gekennzeichnet, dass** wenigstens die einander gegenüberliegenden Flächen der gegenüberliegenden Wandteile (25, 26) des Klemmkörpers einen Friktionskoeffizienten von wenigstens 0,02, vorzugsweise wenigstens 0,03 hat.

11. Ein Verfahren zur Verwendung des Drehers (1) für einen medizinischen Führungsdraht nach einem der vorangehenden Ansprüche 1 - 10, wobei das Verfahren die folgenden Schritte aufweist:
- Positionieren eines Führungsdrahts (2) in der Durchgangsbohrung (4) eines länglichen Körpers (3) und
- Aktivieren der Betätigungsmittel (11) zur Steuerung der Klemmkraft des Klemmkörpers (17) auf den Führungsdraht (12).

12. Ein Verfahren nach Anspruch 11, wobei das Verfahren weiter einen der folgenden Schritte aufweist:
- Verlagern des Drehers (1) in die zweite Position des Klemmkörpers (17) und
- Rotieren des Drehers (1), der mit dem Führungsdraht (2) fixiert ist, in die erste Position des Klemmkörpers (17).

## Revendications

1. Couple de fil guide médical (1) comprenant :
un corps allongé (3) définissant un axe longitudinal (A) et doté de moyens pour recevoir le fil guide (2),
lesquels moyens comprennent un alésage de passage (4) s'étendant à l'intérieur du corps allongé (3) et un corps de serrage (17) pour, en réponse à l'application d'une force externe, dans une première position assurer le couple (1) sur le fil guide (2) et dans une deuxième position, libérer le couple (1) du fil guide (2),
la paroi (9) du corps allongé (3) a une ouverture traversante (10),
des moyens d'actionnement (11) sont agencés dans l'ouverture traversante (10) en contact avec le corps de serrage (17) pour actionner le corps de serrage (17) entre les première et deuxième positions, et où :
l'alésage de passage (4) a une section (18) de surface transversale agrandie et le corps de serrage (17) est prévu dans ladite section (18),
**caractérisé en ce que** le corps de serrage (17) comprend deux parties de paroi parallèles allongées (15, 16) divergeant l'une de l'autre vers l'ouverture traversante (10) afin de définir un espace (27) pour recevoir le fil guide (2).

2. Couple de fil guide médical (1) selon la revendication 1, **caractérisé en ce que** les parties de paroi parallèles allongées (25, 26) opposées sont interconnectées à l'opposé de l'ouverture traversante (10).

3. Couple de fil guide médical (1) selon les revendications 1 ou 2, **caractérisé en ce que** le corps de serrage (17) comprend en outre des moyens de ressort (28) pour forcer les parois allongées (25, 26) du corps de serrage (17) l'une vers l'autre.

4. Couple de fil guide médical (1) selon la revendication 3, **caractérisé en ce que** les moyens de ressort (28) sont un ressort en forme de U (28) qui met en prise au moins une partie du corps de serrage (17) à l'opposé de l'ouverture traversante (10).

5. Couple de fil guide médical (1) selon l'une quelconque des revendications 3 ou 4, **caractérisé en ce que** les moyens de ressort (28) font partie intégrante du corps de serrage (17).

6. Couple de fil guide médical (1) selon l'une quelconque des revendications précédentes 1 à 5, **caractérisé en ce que** les moyens d'actionnement (11) sont un bouton d'actionnement (14) s'étendant à travers l'ouverture traversante (10).

7. Couple de fil guide médical (1) selon la revendication 6, **caractérisé en ce que** le bouton d'actionnement est un moyen de cale (14).

8. Couple de fil guide médical (1) selon l'une quelconque des revendications précédentes 1 à 7, **caractérisé en ce que** les moyens d'actionnement (11) sont déplaçables dans au moins l'une des directions (F, B) sensiblement le long de l'axe longitudinal du corps allongé et sensiblement perpendiculaire (P₁, P₂) à l'axe longitudinal (A) du corps allongé (3).

9. Couple de fil guide médical (1) selon l'une quelconque des revendications précédentes 1 à 8, **caractérisé en ce que** le corps allongé (3) a une partie de préhension (7) de diamètre réduit, de préférence ladite partie de préhension (7) a une surface extérieure irrégulière.

10. Couple de fil guide médical (1) selon l'une quelconque des revendications précédentes 1 à 9, **caractérisé en ce qu'**au moins la face opposée des parties de paroi opposées (25, 26) du corps de serrage (17) a un coefficient de friction d'au moins 0,02, de préférence d'au moins 0,03.

11. Procédé pour utiliser le couple de fil guide médical (1) selon l'une quelconque des revendications précédentes 1 à 10, dans lequel le procédé comprend les étapes consistant à :
positionner un fil guide (2) dans l'alésage de passage (4) du corps allongé (3), et
activer les moyens d'actionnement (11) pour contrôler la force de serrage du corps de serrage (17) sur le fil guide (12).

12. Procédé selon la revendication 11, dans lequel le procédé comprend en outre l'une quelconque des étapes consistant à déplacer le couple (1) dans la deuxième position du corps de serrage (17) et faire tourner le couple (1) fixé sur le fil guide (2) dans la première position du corps de serrage (17).
